# EUROPEAN PATENT SPECIFICATION

(11) **EP 1 690 515 B1**
(45) Date of publication and mention of the grant of the patent: **30.07.2008**
(21) Application number: 06101425.4
(22) Date of filing: 08.02.2006
(51) Int. Cl.: A61F 2/24

(54) **Cardiac-valve prosthesis**
Herzklappenprothese
Prothèse de valvule cardiaque

(30) Priority: 10.02.2005 IT TO20050074
(43) Date of publication of application: 16.08.2006
(60) Divisional application: 08150075.3
(73) Proprietor: SORIN BIOMEDICA CARDIO S.R.L., 13040 Saluggia (Vercelli) (IT)
(72) Inventor: BERGAMASCO, Giovanni, 10137, Torino (IT); BURRIESCI, Gaetano, WC1E 7 JE, London (GB); RIGHINI, Giovanni, 10034, Chivasso (Torino) (IT); STACCHINO, Carla, 10132, Torino (IT)
(74) Representative: Bosotti, Luciano

(56) References cited:
- EP-A- 1 570 809
- WO-A-20/05046528
- NL-C2- 1 017 275
- US-A- 4 692 164
- US-A1- 2005 137 688

## Description

The present invention relates to cardiac-valve prostheses.

The invention has been developed with particular attention paid to cardiac-valve prostheses that can be implanted, for example, via percutaneous route, typically via catheterism as described in the document No. US-A-2002/0042651, or resorting to thoracic-microsurgery and sutureless techniques. The preferential reference to said contexts of use must not, however, be interpreted as in any way limiting the scope of the invention.

In the course of the last few years there has begun to be considered to an ever-increasing extent the possibility of using, as an alternative to traditional cardiac-valve prostheses, whether of a mechanical type or of a biological type, valves designed to be implanted via percutaneous route (the so-called "percutaneous valves").

Implantation of a percutaneous valve (or implantation using thoracic-microsurgery techniques) is in fact a far less invasive act than the surgical operation required for implanting a cardiac-valve prosthesis of a traditional type.

Even though it is a relatively recent technology, a rather extensive and articulated literature has already been dedicated to percutaneous valves. In particular, the patent literature on the subject can be viewed as organized according to a certain number of fundamental categories.

A first category of the technique has concentrated on the development of a percutaneous valve prosthesis consisting substantially of a single structure comprising an anchorage element - which is able to support and fix the valve prosthesis stably in the implantation position - and prosthetic valve elements stably connected to the anchorage element - generally in the form of leaflets or flaps, which are able to regulate the blood flow according to criteria aimed at reproducing natural blood flow in the most faithful way possible.

The anchorage element can present a wide range of conformations, presenting, for example, in the form of an annular structure consisting of one or more rings (US-A-4 056 854, US-A-5 370 685, US-A-5 545 214, US-A-5 824 064, and WO-A-02 41 789), of a so-called "safety pin" (US-A-4 994 077), of an expandable stent with an open-work or reticular tubular wall (US-A-5 411 552, EP-A-0 850 607, DE-A-19 546 692, US-A-5 840 081, US-A-5 855 597, US-A-5 855 601, US-A-5 957 949, WO-A-00 41652, US-B1-6 168 614, US-A-2001/0 007 956, US-A-2001/0 010 017, WO-A-01 76510, US-B1-6 454 799, US-A-6 482 228, WO-A-03 003943), or again of an expandable stent consisting of a sheet or film wound in an optionally open-worked spiral (US-A-5 925 063, WO-A-02 076348).

Prosthetic-valve elements usually consist of biocompatible material and generally have a structure of a membrane or leaflet type which enables connection thereof to the anchorage element according to various known techniques. There has, however, also been envisaged the use, instead of valve leaflets, of an open/close element in the form of a disk, as in the document No. US-A-4 994 077.

A second category of the technique has instead developed percutaneous cardiac-valve prostheses formed by two separate structures, an anchorage element and a set of prosthetic-valve elements co-ordinating with the anchorage element. The two structures are implantable in succession one after another, with the anchorage element set in position first. An example of said valve prosthesis with so-called "double structure" is represented by a stent in the form of a cage, with a function of support and anchorage of the valve, to which is associated a valve element in the form of an arrest ball, as described in the documents Nos. US-A-5 332 402 and US-A-5 397 351, or of an umbrella, as described in US-A-5 332 402.

Again, the patent literature describes elements of support and/or anchorage for valve prostheses - which can be used with valve prostheses of a different nature - having structures of the stent type, as described in the documents Nos. DE-A-19 857 887 and WO-A-01 62189 or of an annular type, as described in the documents Nos. US-A-5 980 570, EP-A-1 088 529 and WO-A-01 64137.

Other patent documents concerning percutaneous cardiac-valve prostheses and corresponding methods of implantation are the documents Nos. US-A-3 671 979, US-A-4 777 951 and US-A-5 954 766.

In general, in the fabrication of cardiac valves, and in particular in the fabrication of percutaneous cardiac valves that are to be implanted in the aortic position, it is necessary to reconcile various needs, which are in effect in contrast with one another.

In the first place, it is important to ensure firm anchorage of the valve in the implantation position. In fact, once implanted, the valve must not run the risk of being displaced (dislodged) with respect to said implantation position as a result of the hydraulic thrust exerted by the blood flow.

In the second place, it is important to ensure the tightness of the artificial duct obtained with the valve with respect to the flow duct generically defined by the natural annulus and by the root of the aorta, preventing undesirable ports for flow of the blood from possibly being set up on the outside of the structure of the valve.

Again, as in the case of all cardiac-valve prostheses, it is desirable to have available a structure of prosthetic valve leaflets that can reproduce as faithfully as possible the operation of natural valve leaflets, this as regards minimization of the loss of head induced on the blood flow and as regards the possibility of preventing lines of flow of the blood through the prosthesis from being set up that might depart, as regards their pattern, from the pattern of the lines of flow characteristic of natural blood flow.

Furthermore, it should be taken into account that the methods of implantation of valves via percutaneous route or by means of thoracic microsurgery are very frequently irrespective of the effective removal of the natural valve leaflets. Very often it is in fact simply envisaged that the cardiac valve is in some way introduced in a position corresponding to the natural annulus and splayed out *in situ* by simply divaricating definitively the natural valve leaflets, the functionality of which is destined to be replaced by the valve leaflets of the prosthetic valve.

The state of the art under Art. 54.3 EPC also includes EP-A-1 570 809. More specifically, the invention relates to a cardiac-valve prosthesis according to the preamble of claim 1, which is known, e.g. from US-A-4 692 164.

The purpose of the present invention is to provide a cardiac valve capable of meeting in an excellent way the requirements indicated previously, with particular reference to the possible implantation via percutaneous route or by means of thoracic microsurgery.

According to the present invention, said purpose is achieved thanks to a cardiac-valve prosthesis having the characteristics referred to specifically in claim 1.

The valve thus obtained is suited in a particularly advantageous way to being implanted in the so-called sinuses of Valsalva.

In the currently preferred embodiment, the valve according to the invention is made so that the entire armature of the valve, or at least the anchorage parts, adhere to the native walls of the implantation site, without interfering with the blood flow, which thus remains practically free. In a preferred way, the anchorage portions moreover have appropriate slits that prevent their interference with the coronary ostia. In said currently preferred embodiment, the anchorage portions and the portions of functional support of the armature can constitute either different parts of a single structure or parts that are structurally distinct from one another.

Super-elastic materials can be used in order to obtain a structure able to self-recover the expanded geometry, once the prosthesis is deployed within the implant position. The entire armature of the valve, or at least the anchorage parts, can be made of re-absorbable material, whereas the valve leaflets can be made of biological and/or synthetic tissues, in part colonizable or re-absorbable. In this way, it is possible to obtain anchorage of the device during the period necessary for integration of the valve prosthesis with the physiological tissues of the anatomical site of implantation. Subsequently, there is dissolution of the artificial structure that enables initial anchorage. Amongst the various advantages linked to this solution to be emphasized is the creation of the ideal conditions for a possible prosthetic re-implantation. Once again in a preferred way, the armature can contain anchorage formations or portions of the supporting structure of the valve flaps made at least partially of shape-memory material (e.g., Nitinol) that enable creation or regulation of the anchorage, i.e., regulation of the modalities and the magnitude of splaying-out of the valve leaflets through control of the memory of the shape-memory material (e.g., by controlling its temperature), according to a mechanism similar to what is described in the document No. EP-A-1 088 529.

The invention will now be described, purely by way of non-limiting example, with reference to the annexed plate of drawings, in which:
- Figure 1 is a general perspective view of a cardiac-valve prosthesis of the type described herein; and
- Figures 2 to 10 illustrate different embodiments of the solution described herein.

In the figures of the annexed plate of drawings, the reference number 1 designates as a whole a cardiac-valve prosthesis (in brief "cardiac valve"), which can be implanted percutaneously or resorting to techniques of thoracic microsurgery, or else of implantation of a "sutureless" type.

For a circumstantiated description of the meaning attributed to said terms and of the valve solutions already proposed in the past the reader is referred to the introductory part of the present description. What has been said also applies as regards the general illustration of the potential advantages linked to the use of cardiac-valve prostheses of this nature and of the corresponding modalities of implantation. It is consequently superfluous herein to proceed again to a detailed exposition of said ideas, which are certainly to be deemed known. The same applies also to a fair extent, with the exception of the elements that will be recalled specifically in what follows, as regards the details of implementation of a strictly technological nature (choice and treatment of the materials, etc.).

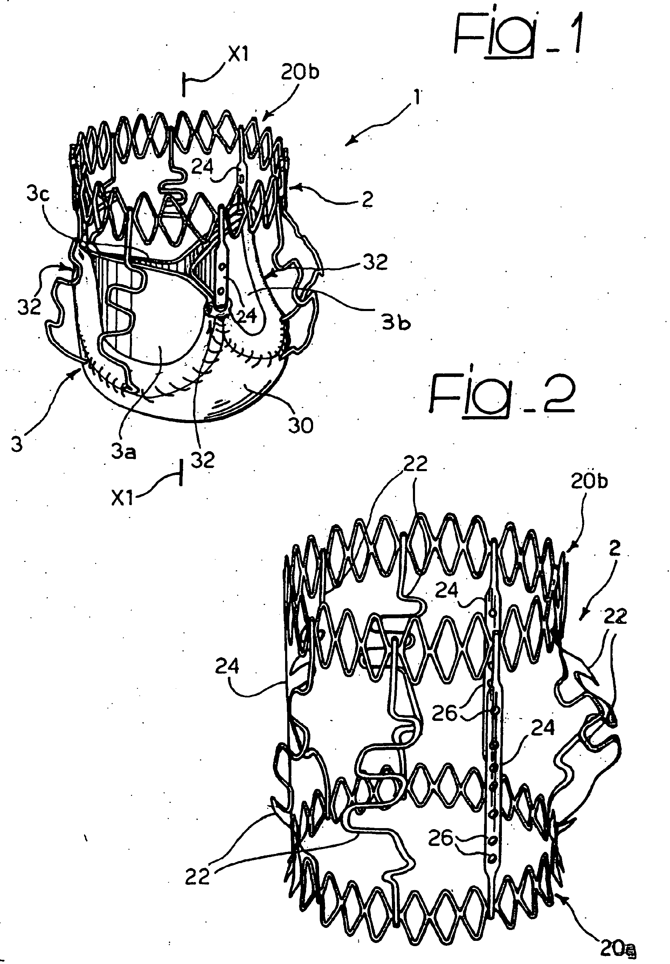

Essentially, the valve 1 represented in Figure 1 consists substantially of two elements, namely:
- an armature 2, having the characteristics that emerge more clearly from the representation of Figures 2 to 7; and
- a set of leaflets or valve sleeve 3, applied on the armature 2 and comprising three valve leaflets 3a, 3b, 3c.

As may be seen more clearly in Figures 2 to 7, the armature 2 of the valve has a general cage-like structure, with a general symmetry of a cylindrical type about a principal axis X1.

In percutaneous valves the axis X1 is designed to correspond usually to the principal axis of the distal part of the catheter used for implantation of the valve
1. For the present purpose, the axis X1 can be viewed basically as an entity of a purely geometrical nature.

A major characteristic of the solution described herein is represented by the absence of structural elements that can extend in the lumen of flow of the blood.

As regards the construction of the set of leaflets or valve sleeve 3, in the framework of the solution described herein it is possible to resort to various solutions known to the art. For example, techniques for the formation of the valve leaflets, assembly of the valve sleeve and installation thereof on an armature that can be used in the context of the present invention are described, for example, in EP-A-0 133 420, EP-A-0 155 245 and EP-A-0 515 324, the latter document referring, however, to the construction of a cardiac-valve prosthesis of biological tissue of the type commonly referred to as "stentless".

For the present purpose, it will be sufficient to recall that the valve sleeve 3 comprises a base portion 30 with an overall annular pattern, designed to extend from the portion of the valve 1, which, in the implantation site, is in a position proximal with respect to the direction of flow of the blood through the prosthesis (from below upwards, as viewed in Figure 1). Starting from the base portion 30, there extend in an axial direction towards the inside of the structure of the prosthesis 1 three pleat formations 32. The valve leaflets 3a, 3b and 3c extend like a festoon, with a general claw-like conformation, between pairs of formations 32 adjacent to one another.

Each valve leaflet 3a, 3b and 3c has then:
- a fluidodynamically proximal edge with an arched pattern, which extends from the base formation 30 and along two adjacent pleat formations 32; and
- a fluidodynamically distal edge, which extends towards the central orifice of the prosthesis so as to be able to co-operate with the homologous edges of the other valve leaflets.

The terms "fluidodynamically proximal" and "fluidodynamically distal" evidently refer to the direction of free flow of the blood through the prosthesis, a direction that is from below upwards, as viewed in the figures of the annexed plate of drawings.

The valve leaflets 3a, 3b, 3c are able to undergo deformation, divaricating and moving up against the armature 2 so as to enable free flow of the blood through the prosthesis.

When the pressure gradient, and hence the direction of flow, of the blood through the prosthesis tends to be reversed, the valve leaflets 3a, 3b, 3c then move into the position represented in Figure 1, in which they prevent the flow of the blood through the prosthesis. Usually, the valve leaflets 3a, 3b, 3c are made in such a way as to assume spontaneously, in the absence of external stresses, the closed configuration represented in Figure 1.

What has been mentioned above in reference to the valve leaflets 3a, 3b and 3c on the other hand corresponds to information and principles of operation well known to the art and such as not to call for a more detailed description herein.

Passing now to a more detailed examination of the structure of the armature 2 (which is usually made of metal material, such as for example the material commonly referred to as Nitinol), it is possible to note therein the presence of two annular parts 20a, 20b, which usually occupy an end position within the armature 2.

In the site of implantation of the prosthesis, the annular parts 20a and 20b are designed to be located, respectively, upstream and downstream of the sinuses of Valsalva.

When the prosthesis 1 is caused to advance towards the implantation site in a radially contracted condition, the annular parts 20a and 20b are in a condition of minimum diameter in the region of 5-15 mm, according to the technique of implantation for which the prosthesis is designed.

Once they have reached the implantation site, the parts 20a, 20b are made/allowed to expand until they reach their normal expanded condition, with a diameter that ranges typically from 18 to 30 mm.

In order to enable the movement of expansion, the annular parts 20a and 20b are usually made with a mesh structure substantially resembling the mesh structure of a stent for angioplasty. It will be appreciated in fact that the annular parts 20a and 20b are designed to perform a movement of radial expansion (with subsequent maintenance of the radially expanded condition) substantially resembling the movement of expansion *in situ* of an angioplasty stent.

In the example of embodiment illustrated herein (which, it is emphasized, is nothing more than an example) the annular parts 20a and 20b have a rhomboidal-mesh structure. Albeit currently preferred, this solution is not to be deemed in any way imperative: in practice, the parts 20a, 20b can be made with any structure that is able to ensure the necessary functionality.

It will moreover be appreciated that the annular part 20a designed to be located in a position proximal with respect to the flow of the blood through the prosthesis (i.e., on the inflow side of the blood in the prosthesis in the conditions of free flow) usually has a pattern at least slightly flared like an enlarged opening of the flow duct of the blood. This has the function of ensuring a better anchorage to the valve annulus, guaranteeing the perivalvar tightness, and improving the haemodynamics, adapting (i.e., radiusing) the lines of blood flow in the ventricular chamber to the flow tube constituted by the valve sleeve.

The annular parts 20a, 20b are connected to one another by anchorage formations 22 presenting a general arched pattern, projecting towards the outside of the prosthesis. The formations 22 are designed in fact to extend on the outside of the sinuses of Valsalva so as to ensure firm anchorage *in situ* of the prosthesis 1.

With the prosthesis in the radially contracted position adopted for the implantation, the formations 22 are normally maintained in the position recalled towards the central axis X1 of the prosthesis: this occurs for example, via a tubular sheath associated to the implantation catheter). Subsequently, once disengaged from the retention means, the formations 22 assume the arched pattern represented in the figures so as to be able to project (protrude) within the sinuses of Valsalva.

As already discussed, the prosthesis described herein has features that make it suitable to be implanted both in an aortic and in a pulmonary position.

For instance, the sinuses of Valsalva are, in a normal heart, three in number, distributed in an approximately angularly uniform way around the root of the artery distal to the semilunar valve (aortic or pulmonary valve). Accordingly, in the valve illustrated, the formations 22 are three in number (or in three groups) set at an angular distance apart of 120° with respect to the central axis X1 of the prosthesis.

In the exemplary embodiment illustrated, the formations 22 are made in the form of ribbon-like elements that extend according to a general sinusoidal path or serpentine path, with bends or open loops situated on either side with respect to an ideal line extending approximately in the direction of the generatrices of the overall cylindrical shape of the prosthesis.

The above solution is, however, in no way imperative.

In a possible variant embodiment of the invention, the sinusoidal pattern can be obtained with bends or open loops that extend from one side and from the other with respect to a line that extends in a circumferential direction with respect to the prosthesis. Of course, also as regards the formations 22 it is possible to resort to a mesh structure, for example closed rhomboidal meshes of the same type as the one represented with reference to the parts 20a, 20b, or to simple segments of curve lying in roughly radial planes.

As has already been said, each formation 22 can consist either of a single element or of a plurality of elements that extend in a direction in which they are generically set alongside one another.

The annular parts 20a and 20b, as well as the respective anchorage elements 22, substantially form the basic structure or armature of the prosthesis 1, designed to ensure positioning and anchorage *in situ* of the prosthesis 1 itself.

Associated then to the parts 20a and 20b are further elements, generically designated by 24 in all of Figures 2 to 7, the function of which is to ensure support of the set of leaflets or valve sleeve 3 on the armature of the prosthesis.

In the embodiment represented in Figure 2, the elements 24 are simply represented by three bars of flattened shape that extend as connection of the formations 20a, 20b. The elements 24 are set at an angular distance apart of 120° and are located in a position that is generically intermediate between the anchorage formations 22.

As may at once be appreciated from a comparative examination of Figures 1 and 2, the formations 24 are designed to enable the installation of the valve sleeve 3 in a condition such that the base portion 30 thereof is arranged in general in a position of winding of the angular formation 20a of the armature 2, whilst each of the pleat formations or folds 32 in turn embraces one of the elements or formations 24, whilst the valve leaflets 3a, 3b and 3c extend in a festoon, each between two adjacent formations 24. The general apertured structure both of the part 20a and of the formations 24 (note the particular holes designated by 26) enables fixing of the valve sleeve 3 on the armature 2, resorting, for example, to suturing stitches applied according to known techniques. In the case where flaps of polymeric materials are used, the flaps can be formed directly on the structure, using techniques such as, for example, dip casting.

In this regard, both the armature 2 and the aforesaid suturing stitches can be advantageously provided with a coating of biocompatible carbon material, which is applied by resorting, for example, to the solutions described in US-A-4 624 822, US-A-4 758 151, US-A-5 084 151, US-A-5 133 845, US-A-5 370 684, US-A-5 387 247, and US-A-5 423 886.

The apertured structure of the supporting formations 24, and of the armature 2 in general, means that the armature does not exert any obtrusive effect, preventing, for example, interference in regard to the coronary ostia.

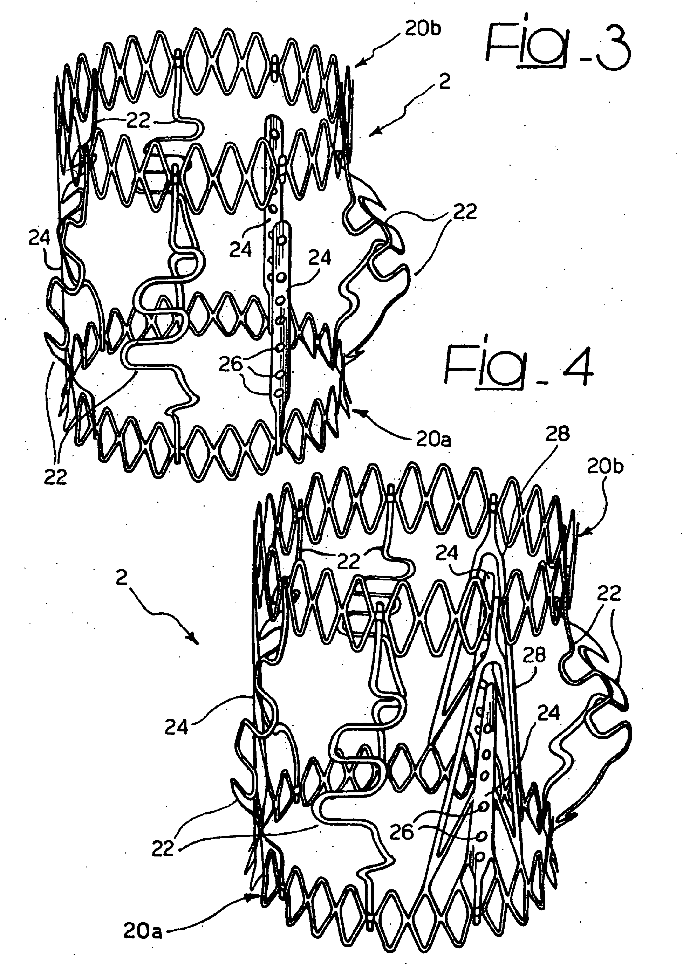

The variant embodiment represented in Figure 3 as a whole resembles the solution represented in Figure 2, with the exception that (in the solution of Figure 3) the formations 24 provided for anchorage of the valve sleeve 3 do not extend completely in bridge-like fashion between the two annular parts 20a and 20b. In the embodiment illustrated in Figure 3, the elements 24 are in fact made in the form of projecting elements that extend in cantilever fashion starting from the annular formation 20a, without reaching the formation 20b. In particular, the length of the aforesaid cantilever formations is determined in such a way as to extend for a length sufficient to enable anchorage of the valve sleeve 3 at the pleat formations 32.

As compared to the solution illustrated in Figure 2, the variant of Figure 3 presents the characteristic of not having portions of the elements 24 in addition to the ones that perform directly the function of support of the valve sleeve.

The embodiment represented in Figure 4 basically reproduces the solution of Figure 3, with the difference represented by the fact that the formations 24 (which also in this case present a cantilever conformation and project in cantilever fashion from the annular part 20a) have associated thereto fork-like structures 28. Each fork-like structure 28 has a root portion connected to the annular part 20b and two prongs that extend on either side with respect to the formations 24 and then connect up to the annular part 20a on opposite sides with respect to the area in which the formation 24 projects in cantilever fashion from the formation 20a.

In this case, the formations 24 have a tapered pattern. Preferably, said tapered pattern is made with a width that decreases gradually moving away from the annular part 20a, and hence in the proximal-to-distal direction with reference to the direction of free flow of the blood through the prosthesis.

This characteristic of tapering can on the other hand be extended to the formations 24 of all the solutions represented in Figures 2 to 4. This extends in practice to all the other characteristics of the formations 24 (and also of the formations 22), which, albeit herein represented as identical to one another in each of the embodiments illustrated, could in actual fact be different from one another, thus envisaging the coexistence, in a single prosthesis, of formations 22 or 24 different from one another, with characteristics drawn from different embodiments amongst the plurality of embodiments illustrated herein.

The foregoing explains among other things why, in the characteristics recited in the claims annexed hereto, reference is made to characteristics that can be identified in "at least one of the formations" 22 or 24.

The solution represented in Figure 4 enables maintenance, with respect to the solution appearing in Figure 3, of a more rigid anchorage structure, in which the expansion of the elements 22 in the sinuses of Valsalva is favoured by the fixed axial dimension of the prosthesis, which is guaranteed by the presence of the fork-like formations 28. However, the formations 24, which have the function of anchorage of the valve sleeve, are maintained flexible and of modulatable stiffness.

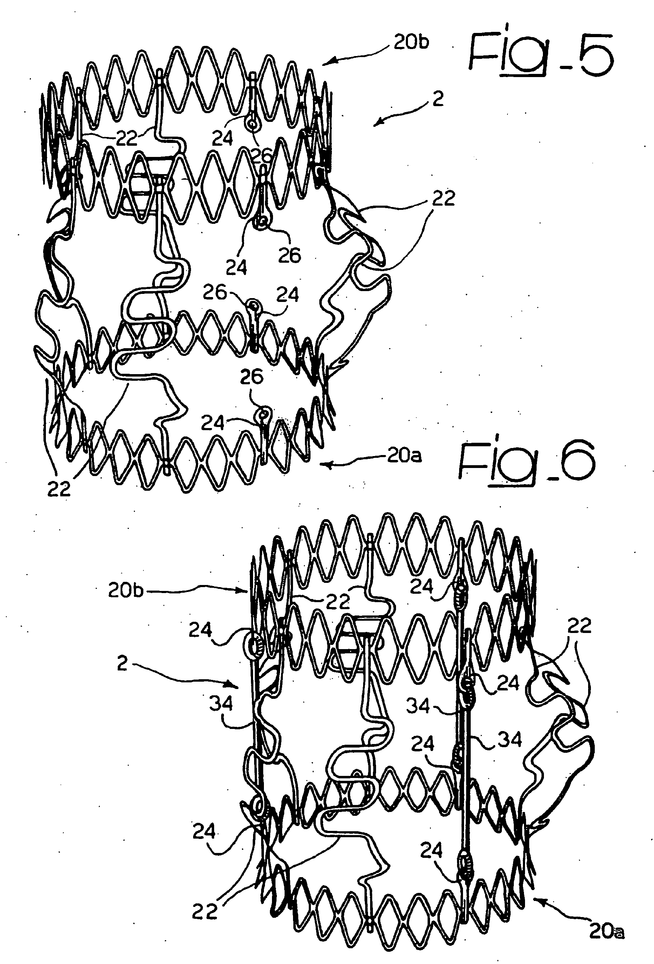

In the variant represented in Figure 5 (as in the variant represented in Figure 6), the formations 24 are provided in positions corresponding to both of the annular parts 20a, 20b. In this case, however, the formations 24 provided for anchorage of the valve sleeve are reduced to small hooking cantilevers, each provided with an eyelet 26.

The eyelets in question can be used directly for passing and tying the wires that extend from the valve sleeve 30.

In the solution represented in Figure 6, it is envisaged that the formations 24, arranged in pairs in positions facing the parts 20a, 20b, will be connected by flexible elements having a filiform pattern, designated by 34. The flexible material of the elements 34 can be constituted, for example by biocompatible metal alloys (e.g., Nitinol) or by materials of a polymeric nature that are suited to applications in the field of implantations (e.g., acetal resins).

From the geometrical standpoint, the overall configuration then resembles the one represented in Figure 2. The difference lies in the fact that, whereas the formations 24 represented in Figure 2 are as a whole stiff (of course taking into account the intrinsic flexibility of the material that constitutes them), the elements 34 in Figure 6 can be made of a filiform material with high flexibility.

Consequently, the embodiment represented in Figure 6 envisages the presence of at least one pair of homologous supporting formations 24, which extend in cantilever fashion towards one another, starting from one and the other of the annular parts 20a and 20b. The aforesaid homologous supporting formations 24 are flexibly connected to one another by the supporting element 34.

The fact of resorting to the solution represented in Figure 6 hence enables the configuration for hooking of the valve sleeve 3 to the armature 2 of the prosthesis to be rendered elastic/flexible and renders the extent of the stretches 22 independent of that of the portions 24, thus enabling a greater elasticity of design.

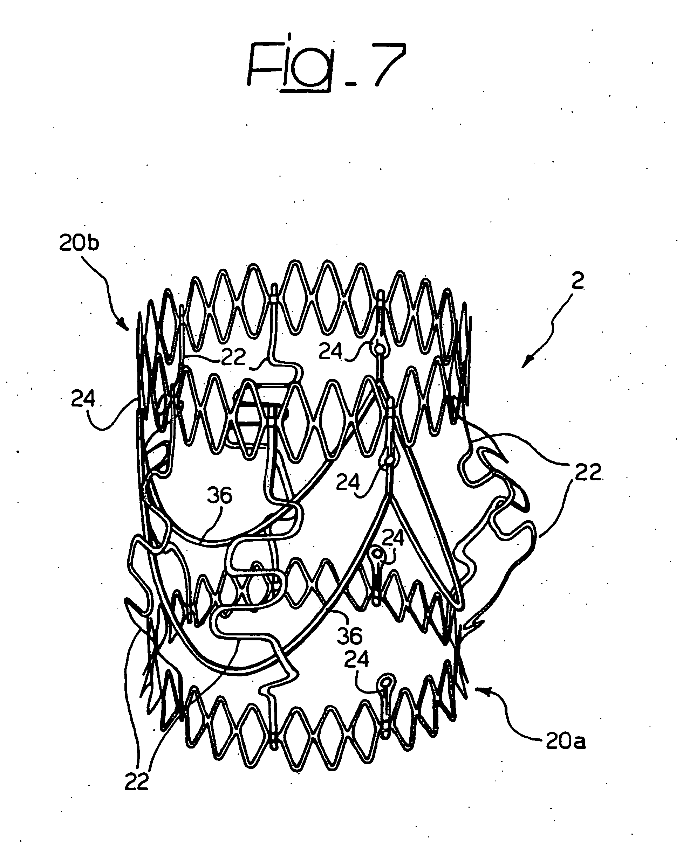

Finally, the further variant represented in Figure 7 is conceptually akin to the solution illustrated in Figure 6, with the difference represented by the fact that in the solution of Figure 7 the elements of flexible material associated to the formations 24 do not extend as connections of mutually facing pairs of formations 24.

Instead, in the embodiment represented in Figure 7, the formations made of rigid or flexible material, designated by 36, extend according to a general festoon-like or catenary path between the formations 24 associated with the formation 20b.

In this case, there is then present at least one pair of combined supporting formations 24, which extend in cantilever fashion starting from one of the annular parts 20a and 20b (in particular, it is the part 20b). The aforesaid supporting formations 24 are connected to one another by a festoon-like supporting element 36, which is able to support one of the valve leaflets 3a, 3b, 3c.

It will be appreciated in fact that the aforesaid festoon-like or catenary pattern is designed to copy the homologous pattern of the proximal edges of the valve leaflets 3a, 3b and 3c, defining the profile of the edge for anchorage of the functional flaps and, possibly, enabling connection by suture of the aforesaid proximal edges of the valve leaflets to the festoon-like elements 36.

The above solution enables use of extremely simple valve sleeves, assigning the formation of the profile of the functional flaps to the catenary formations 36.

The solution described herein enables, in the final position of implantation, the entire armature of the valve, or at least the anchorage parts, to adhere to the native walls of the implantation site, without interfering with the blood flow, which thus remains practically free.

The anchorage portions moreover have an apertured structure (for example, appropriate slits), which prevents interference with the coronary ostia.

The anchorage portions and the portions of functional support of the armature can constitute either different parts of a single structure or parts that are structurally distinct from one another. The entire armature, or at least the anchorage parts, can be made of re-absorbable material, whereas the valve sleeve can be constituted by biological and/or synthetic tissues, which are in part colonizable or re-absorbable.

Again, the armature can contain anchorage formations made at least partially of shape-memory material (e.g., Nitinol), which enable creation or regulation of the anchorage through the control of the memory of the shape-memory material (e.g., controlling its temperature).

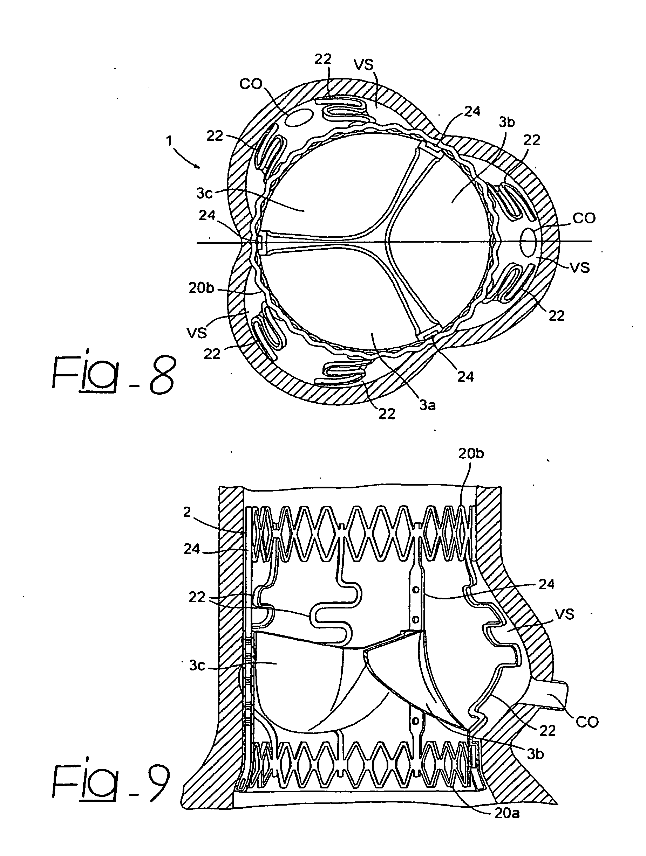

Figures 8 and 9 illustrate plan and cross-sectional views, respectively, of the prosthesis 1 in its implanted state in an aortic valve replacement, according to an embodiment of the invention. As shown, and as discussed in detail above, the prosthesis 1 can be implanted such that the annular elements 20a and 20b occupy positions proximal and distal, respectively, of the Valsalva sinuses VS, with the flared proximal end of the annular member 20a forming the proximal entrance of the lumen defined by the armature 2 of the prosthesis 1. In the illustrated embodiment, the anchor members 22 can be arranged in three pairs positioned relative to the sinuses of Valsalva such that the radially-projecting portion of each of the anchor members 22 projects into the respective sinus of Valsalva and engages the aortic wall therein. As further shown, the anchor members 22 of each pair can be positioned on opposite sides of the coronary ostia CO in the respective sinuses of Valsalva. Additionally, as discussed above and shown in Figures 8 and 9, the serpentine or otherwise generally apertured structure of the anchor members 22 substantially avoids interference with the coronary ostia CO. Finally, as can be seen from Figures 8 and 9, the valve leaflets 3a, 3b, 3c can be positioned within the lumen for blood flow formed by the annular elements 20a, 20b, with the support members 24 extending into the lumen by a minimal amount.

The armature 2 of the prosthesis 1, according to one embodiment, is manufactured by first cutting a blank part from a tube of a biocompatible metal (e.g., Nitinol, or a cobaltum-chromium alloy) having an outer diameter which is at an intermediate size between the fully radially contracted and the fully expanded device dimensions. For example, the tube may have an outer diameter of between about 8 mm to about 14 mm. In one embodiment, the tube has a diameter of about 10 mm. In one embodiment, the tube wall may vary between about 0.3 mm to about 0.5 mm, depending on the required stiffness required and the size of the prosthesis 1.

In one embodiment, the final dimension and shape of the framework is achieved by a sequence of expansion cycles. A specific heat treatment is applied after each expansion cycle to homogenize and stress relieve the material, which allows the shape and properties of the structure of the armature 2 to be set. Although the number of forming steps may vary among devices, for the geometries described above with respect to the present invention, and using Nitinol for the tube blank, an exemplary number of forming steps is around three. Among these steps, the first two provide the final diameter of the annular elements 20a, 20b. For example, if the fully-expanded diameter for implantation is 23 mm, the final cylindrical shape of the armature 2 can be achieved using a tube blank of about 10 mm in diameter, a first expansion from about 10 mm to about 18 mm, and a second expansion from about 18 mm to about 23 mm. Optionally, the final diameter can be made slightly larger (e.g. about 25 mm in the previous example) in order to oversize the armature 2 with respect to the physiological annulus, thus imparting a radial force to the wall of the annulus at the nominal implant diameter.

The third forming step is aimed to impart the radially-extending shape of the anchor members 22 such that they will fit and anchor within the Valsalva sinuses. The corresponding heat treatment, according to one embodiment, includes exposing the deformed armature 2 to a temperature from about 480 °C to about 550 °C, for a time ranging from about 5 minutes to about 30 minutes, depending on the desired final transformation temperature. For example, in order to obtain a super-elastic behavior at 37°C (the normal working condition in human body) the heat treatments subsequent to the two initial expansion steps may be performed at about 480°C for a time of about 9 minutes, and the final heat treatment (after the third expansion) is performed at about 500°C for a time of about 9 minutes.

After the forming process is complete, the armature 2 may undergo one or more surface treatments, for example, sandblasting and electropolishing, to provide a sufficiently smooth surface and to remove the shallow defects. The armature 2 may thereafter be finally exposed to a carbon coating process in order to improve its hemocompatibility.

As shown in Figures 8-9, for an aortic valve replacement, the final geometrical shape of the armature 2 will generally approximate the physiological shape and dimension of the aortic root, such that the anchor members 22 generally conform to the walls of the respective Valsalva sinuses VS.

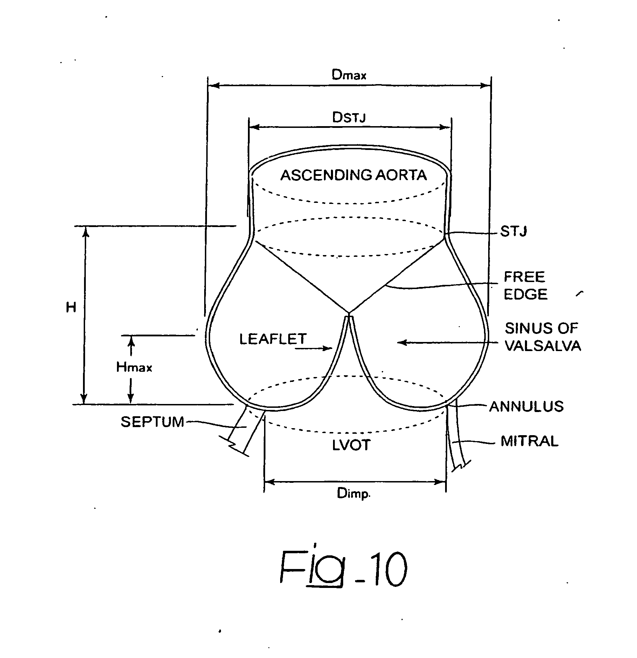

Figure 10 shows a schematic cross sectional view of an implantation site for an aortic valve replacement. Referring to Figure 10, exemplary proportions of the relevant features at the implantation site for an aortic valve replacement are as follows (assuming the annulus diameter Dᵢₘₚ (implanting diameter) equal to 1):

| | Approximate Minimum | Approximate Maximum |
|---|---|---|
| Height of Valsalva sinuses (H): | 0.8 | 1 |
| Max. diameter of Valsalva sinuses (Dmax): | 1.3 | 1.7 |
| Distance between Valsalva max. diameter and basic annulus plane (Hmax): | 0.3 | 0.5 |
| Diameter at the sino-tubular junction (Dstj): | 0.8 | 1.4 |

According to one exemplary embodiment, H is about 0.9, Dmax is about 1.5, Hmax is about 0.35, and Dstj is about 1.2.The commissural points of the elastic collapsible valve 3 are mounted to the armature 2 (e.g., by attachment to the support members 24) such that the valve leaflets 3a, 3b, and 3c can fold and expand together. The valve 3, including the valve leaflets 3a, 3b, 3c, can be, for example, a glutaraldehyde fixed pericardium valve which has three cusps that open distally to permit unidirectional blood flow.

In one embodiment, the valve member may use two pericardium sheets. The first sheet forms the three moving cusps, the second sheet coats part of the armature 2 surface so that there is no contact between the armature 2 and the valve leaflets avoiding the risk of abrasion due to repeated impact against the metallic material of the armature 2. In addition, this second sheet redistributes the stress applied by blood pressure on the prosthetic leaflets, avoiding the risk of stress concentration.

The two sheets of pericardium may be stitched together flat using suture thread coated with a film of biocompatible material, and then close in a cylindrical shape. The type of stitch used may be varied to accommodate the directional differences in the forces exerted at each point of the suture, to ensure that the stitches themselves don't become the origin of fatigue fracture lines. The two sheets may be stitched together in a flat position so when the leaflets open they recover their original cylindrical configuration, forming a cylindrical duct.

The elastically collapsible valve sleeve 3 can be mounted on the armature 2 by means of a number of suture stitches. Both of the sheets are useful for attaching the valve sleeve 3 to the armature 2 by stitching.

The valve member can use a tissue fixation and shaping of the leaflets 3a, 3b, 3c by means of a fluidic, atraumatic system with chemicals useful for cross-linking and then may be exposed to a detoxification post treatment to increase long-term performance. An additional pericardium sheet 30 can be positioned in correspondence with the inflow annular element 20a in a cylindrical fashion with the purpose of improving the sealing capability of the prosthetic device with respect to the implant annulus.

Of course, without prejudice to the principle of the invention, the details of construction and the embodiments may vary widely with respect to what is described and illustrated herein, without thereby departing from the scope of the present invention, as defined by the claims that follow.

## Claims

1. A cardiac-valve prosthesis (1), comprising:
- an armature (2) for anchorage of the valve prosthesis at an implantation site, said armature (2) defining a lumen for passage of the blood flow; and
- a set of prosthetic valve leaflets (3a, 3b, 3c), which are supported by said armature (2) and are able to move, under the action of the blood flow, in a radially divaricated condition to enable the flow of the blood through said lumen in a first direction, and in a radially contracted condition, in which said valve leaflets (3a, 3b, 3c) co-operate with one another and block the flow of the blood through the prosthesis (2) in the direction opposite to said first direction, wherein said armature (2) comprises:
- two annular parts (20a, 20b) connected by connection formations (22), which extend as connection of said annular parts (20a, 20b), with the capacity of projecting radially with respect to the prosthesis (1); and
- supporting formations (24) for said set of leaflets (3), said formations being carried by at least one of said annular parts (20a, 20b) so as to leave substantially disengaged said lumen for passage of the blood,**characterized in that:**
- said annular parts (20a, 20b) have a structure which can expand from a radially contracted condition of advance towards the site of implantation of the prosthesis to a radially expanded condition, in which the prosthesis (1) is withheld in the implantation site, and
- said connection formations (22) have an arched pattern arched towards the outside of the prosthesis (2), said connection formations constituting anchorage formations to ensure anchorage of the prosthesis at the implantation site.

2. The prosthesis according to Claim 1, **characterized in that** said annular parts (20a, 20b) have a mesh structure.

3. The prosthesis according to Claim 2, **characterized in that** said annular parts (20a, 20b) have a rhomboidal-mesh structure.

4. The prosthesis according to any one of the preceding claims, **characterized in that** at least one of said annular parts (20a, 20b) has a general flared pattern.

5. The prosthesis according to any one of the preceding claims, **characterized in that** said anchorage formations (22) are angularly distributed in three sets around the prosthesis (1) so as to be able to extend into the sinuses of Valsalva.

6. The prosthesis according to any one of the preceding claims, **characterized in that** at least one of said anchorage formations (22) has a serpentine pattern.

7. The prosthesis according to any one of the preceding claims, **characterized in that** at least one of said supporting formations (24) extends like a bridge between said annular parts (20a, 20b).

8. The prosthesis according to any one of Claims 1 to 7, **characterized in that** at least one of said supporting formations (24) has an apertured structure (26).

9. The prosthesis according to any one of Claims 1 to 8, **characterized in that** at least one of said supporting formations (24) extends in cantilever fashion from at least one of said annular parts (20a, 20b).

10. The prosthesis according to any one of Claims 1 to 9, **characterized in that** at least one of said supporting formations (24) has a tapered pattern.

11. The prosthesis according to Claim 10, **characterized in that** at least one of said supporting formations (24) has a tapered pattern with a width that decreases in the proximal-to-distal direction with reference to the direction of free flow of the blood through the prosthesis.

12. The prosthesis according to any one of the preceding claims, **characterized in that** at least one of said supporting formations. (24) has associated thereto a fork-like structure (28), said fork-like structure (28) having a root portion connected to one (20b) of said annular parts (20a, 20b) and two prongs that extend from either side with respect to said at least one said supporting formations (24) and then connect up to the other (20a) of said annular parts (20a, 20b).

13. The prosthesis according to Claims 9 and 12, taken in combination, **characterized in that** said two prongs connect up to the other (20a) of said annular parts (20a, 20b) on opposite sides with respect to the area from which said at least one of said supporting formations (24) projects in cantilever fashion from said other (20a) of said annular parts (20a, 20b).

14. The prosthesis according to any one of Claims 1 to 8, **characterized in that** it comprises at least one pair of homologous supporting formations (24), which extend in cantilever fashion towards one another, starting from the one (20a, 20b) and the other (20b, 20a) of said annular parts (20a, 20b), respectively.

15. The prosthesis according to Claim 14, **characterized in that** said homologous supporting formations (24) are flexibly connected to one another by a supporting element (34).

16. The prosthesis according to any one of Claims 1 to 10, **characterized in that** it comprises at least one pair of combined supporting formations (24), which extend in cantilever fashion, starting from one (20b) of said annular parts (20a, 20b), said combined supporting formations (24) being connected to one another by a festoon-like supporting element (36), which is able to support one of said valve leaflets (3a, 3b, 3c).

17. The prosthesis according to any one of the preceding claims, **characterized in that** said anchorage formations (22) and said supporting formations (24) form different parts of a single armature structure (2).

18. The prosthesis according to any one of the preceding Claims 1 to 15, **characterized in that** said anchorage formations (22) and said supporting formations (24) form parts that are structurally distinct from one another.

19. The prosthesis according to any one of the preceding claims, **characterized in that** said armature (2) is made, at least limitedly to said anchorage parts (22), of re-absorbable material.

20. The prosthesis according to any one of the preceding claims, **characterized in that** said anchorage formations (22) are made, at least in part, of shape-memory material.

21. The prosthesis according to any one of the preceding claims, **characterized in that** said valve leaflets (3a, 3b, 3c) are made of materials selected out of the group consisting of biological tissues, synthetic tissues, colonizable tissues and re-absorbable tissues.

## Patentansprüche

1. Eine Herzklappenprothese (1) mit
- einer Verankerung (2), um die Ventilprothese an einem Implantationsort zu verankern, wobei die Verankerung (2) ein Lumen für den Durchtritt des Blutstroms definiert; und
- einem Satz von prothetischen Klappenblättern (3a, 3b, 3c), welche durch die Verankerung (2) getragen werden und in der Lage sind, sich unter der Wirkung des Blutstroms in einem radial aufgeweiteten Zustand, um den Strom des Blutes durch das Lumen in einer ersten Richtung zu ermöglichen, und in einen radial kontrahierten Zustand, in welchem die Klappenblätter (3a, 3b, 3c) zusammenwirken und den Blutstrom durch die Prothese (2) in der zu der ersten Richtung entgegengesetzten Richtung blokkieren, zu bewegen, wobei die Verankerung (2) umfasst:
- zwei ringförmige Teile (20a, 20b), welche durch Verbindungsgebilde (22) verbunden sind, die sich als Verbindung der ringförmigen Teile (20a, 20b) erstrecken mit der Möglichkeit, in Bezug auf die Prothese (1) radial vorzustehen; und
- Traggebilde (24) für den Satz von Blättern (3), wobei die Gebilde durch wenigstens eines der ringförmigen Teile (20a, 20b) in der Weise getragen werden, dass das Lumen für den Durchtritt von Blut im Wesentlichen unbeeinträchtigt bleibt,
**dadurch gekennzeichnet, dass**:
- die ringförmigen Teile (20a, 20b) eine Struktur haben, welche aus einem radial kontrahierten Zustand der Vorwärtsbewegung in Richtung auf den Implantationsort der Prothese in einen radial expandierten Zustand expandieren kann, in welchem die Prothese (1) an dem Implantationsort zurückgehalten wird, und die Verbindungsgebilde (22) eine Bogenform besitzen, welche in Richtung der Außenseite der Prothese (2) gebogen ist, wobei die Verbindungsgebilde Verankerungsgebilde darstellen, um die Verankerung der Prothese an dem Implantationsort sicherzustellen.

2. Die Prothese nach Anspruch 1, **dadurch gekennzeichnet, dass** die ringförmigen Teile (20a, 20b) eine Maschenstruktur besitzen.

3. Die Prothese nach Anspruch 2, **dadurch gekennzeichnet, dass** die ringförmigen Teile (20a, 20b) eine rhombusförmige Maschenstruktur besitzen.

4. Die Prothese nach einem der vorherigen Ansprüche, **dadurch gekennzeichnet, dass** wenigstens eines der ringförmigen Teile (20a, 20b) eine sich konusförmig erweiternde Grundform hat.

5. Die Prothese nach einem der vorherigen Ansprüche, **dadurch gekennzeichnet, dass** die Verankerungsgebilde (22) in drei Sätzen um die Prothese (1) winklig verteilt sind, so dass sie in der Lage sind, sich in die Sinus der Valsalva zu erstrecken.

6. Die Prothese nach einem der vorherigen Ansprüche, **dadurch gekennzeichnet, dass** wenigstens eines der Verankerungsgebilde (22) eine Serpentinenform besitzt.

7. Die Prothese nach einem der vorherigen Ansprüche, **dadurch gekennzeichnet, dass** wenigstens eines der Traggebilde (24) sich wie eine Brücke zwischen den ringförmigen Teilen (20a, 20b) erstreckt.

8. Die Prothese nach einem der Ansprüche 1 bis 7, **dadurch gekennzeichnet, dass** wenigstens eines der Traggebilde (24) eine mit Öffnungen versehene Struktur (26) hat.

9. Die Prothese nach einem der Ansprüche 1 bis 8, **dadurch gekennzeichnet, dass** wenigstens eines der Traggebilde (24) in der Weise eines Auslegers von wenigstens einem der ringförmigen Teile (20a, 20b) abragt.

10. Die Prothese nach einem der Ansprüche 1 bis 9, **dadurch gekennzeichnet, dass** wenigstens eines der Traggebilde (24) eine geneigte Form hat.

11. Die Prothese nach Anspruch 10, **dadurch gekennzeichnet, dass** wenigstens eines der Traggebilde (24) eine geneigte Form hat mit einer Breite, die in der Richtung proximal-nach-distal in Bezug auf die Richtung des freien Stroms von Blut durch die Prothese abnimmt.

12. Die Prothese nach einem der vorherigen Ansprüche, **dadurch gekennzeichnet, dass** wenigstens einem der Traggebilde (24) eine gabelähnliche Struktur (28) zugeordnet ist, wobei die gabelähnliche Struktur (28) einen Basisbereich, der mit einem (20b) der ringförmigen Teile (20a, 20b) verbunden ist, und zwei Zinken, die von jeder Seite in Bezug auf das wenigstens eine Stützgebilde (24) abragen und dann mit dem anderen (20a) der ringförmigen Teile (20a, 20b) verbunden sind.

13. Die Prothese nach den Ansprüchen 9 und 12 in Kombination, **dadurch gekennzeichnet, dass** die beiden Zinken an dem anderen (20a) der ringförmigen Teile (20a, 20b) auf gegenüberliegenden Seiten in Bezug auf den Bereich, von dem das wenigstens eine der Traggebilde (24) in der Weise eines Auslegers von dem anderen (20a) der ringförmigen Teile (20a, 20b) vorsteht, verbunden sind.

14. Die Prothese nach einem der Ansprüche 1 bis 8, **dadurch gekennzeichnet, dass** sie wenigstens ein Paar von homologen Traggebilden (24) besitzt, welche nach Art eines Auslegers in Richtung aufeinander beginnend von dem einen (20a, 20b) und dem anderen (20b, 20a) der ringförmigen Teile (20a, 20b) jeweils abragen.

15. Die Prothese nach Anspruch 14, **dadurch gekennzeichnet, dass** die homologen Traggebilde (24) flexibel miteinander durch ein Stützelement (34) verbunden sind.

16. Die Prothese nach einem der Ansprüche 1 bis 10, **dadurch gekennzeichnet, dass** sie wenigstens ein Paar von kombinierten Traggebilden (24) besitzt, welche sich in der weise eines Auslegers beginnend von einem (20b) der ringförmigen Teile (20a, 20b) erstrecken, wobei die kombinierten Traggebilde (24) miteinander durch ein girlandenförmiges Stützelement (36) verbunden sind, das in der Lage ist, eines der Klappenblätter (3a, 3b, 3c) abzustützen.

17. Die Prothese nach einem der vorherigen Ansprüche, **dadurch gekennzeichnet, dass** die Verankerungsgebilde (22) und die Traggebilde (24) unterschiedliche Teile einer einzigen Verankerungsstruktur (2) bilden.

18. Die Prothese nach einem der vorherigen Ansprüche 1 bis 15, **dadurch gekennzeichnet, dass** die Verankerungsgebilde (22) und die Traggebilde (24) Teile bilden, die strukturell unterschiedlich voneinander sind.

19. Die Prothese nach einem der vorherigen Ansprüche, **dadurch gekennzeichnet, dass** die Verankerung (2) zumindest begrenzt auf die Verankerungsteile (22) aus einem wieder-absorbierbaren Material bestehen.

20. Die Prothese nach einem der vorherigen Ansprüche, **dadurch gekennzeichnet, dass** die Verankerungsgebilde (22) zumindest teilweise aus einem Gedächtnismaterial bestehen.

21. Die Prothese nach einem der vorherigen Ansprüche, **dadurch gekennzeichnet, dass** die Klappenblätter (3a, 3b, 3c) aus Materialien bestehen, die aus der Gruppe bestehend aus biologischen Geweben, synthetischen Geweben, kolonisierbaren Geweben und wieder-absorbierbaren Geweben ausgewählt sind.

## Revendications

1. Prothèse de valve cardiaque (1) comprenant :
- une armature (2) pour l'ancrage de la prothèse de valve dans un site d'implantation, ladite armature (2) définissant une lumière pour le passage de l'écoulement sanguin ; et
- un ensemble de feuillets de prothèse de valve (3a, 3b, 3c) qui sont supportés par ladite armature (2) et qui peuvent se déplacer sous l'effet de l'écoulement sanguin dans un état radialement déployé pour permettre l'écoulement du sang à travers ladite lumière dans une première direction et dans un état radialement contracté dans lequel lesdits feuillets de valve (3a, 3b, 3c) coopèrent les uns avec les autres et bloquent l'écoulement du sang à travers la prothèse (2) dans la direction opposée à ladite première direction, dans laquelle ladite armature (2) comprend :
- deux parties annulaires (20a, 20b) raccordées par des formations de raccord (22) qui s'étendent sous forme de raccord desdites parties annulaires (20a, 20b) et qui sont capables de faire saillie radialement par rapport à la prothèse (1) ; et
- des formations de support (24) pour ledit ensemble de feuillets (3), lesdites formations étant portées par au moins l'une des parties annulaires (20a, 20b) de manière à laisser ladite lumière sensiblement dégagée pour le passage du sang,
**Caractérisée en ce que** :
- lesdites parties annulaires (20a, 20b) ont une structure qui peut être expansée depuis un état radialement contracté d'avancement vers le site d'implantation de la prothèse à un état radialement expansé dans lequel la prothèse (1) est retenue dans le site d'implantation, et
- lesdites formations de raccord (22) ont une configuration en arc cambrée vers l'extérieur de la prothèse (2), lesdites formations de raccord constituant des formations d'ancrage pour assurer l'ancrage de la prothèse dans le site d'implantation.

2. Prothèse selon la revendication 1, **caractérisée en ce que** lesdites parties annulaires (20a, 20b) ont une structure maillée.

3. Prothèse selon la revendication 2, **caractérisée en ce que** lesdites parties annulaires (20a, 20b) ont une structure maillée rhomboïde.

4. Prothèse selon l'une quelconque des revendications précédentes, **caractérisée en ce qu'**au moins l'une lesdites parties annulaires (20a, 20b) a une configuration généralement évasée.

5. Prothèse selon l'une quelconque des revendications précédentes, **caractérisée en ce que** lesdites formations d'ancrage (22) sont réparties angulairement en trois ensembles autour de la prothèse (1) de manière à pouvoir s'étendre dans les sinus de Valsalva.

6. Prothèse selon l'une quelconque des revendications précédentes, **caractérisée en ce qu'**au moins l'une desdites formations d'ancrage (22) a une configuration en serpentin.

7. Prothèse selon l'une quelconque des revendications précédentes, **caractérisée en ce qu'**au moins l'une desdites formations de support (24) s'étend comme un pont entre lesdites parties annulaires (20a, 20b).

8. Prothèse selon l'une quelconque des revendications 1 à 7, **caractérisée en ce qu'**au moins l'une desdites formations de support (24) a une structure ajourée (26).

9. Prothèse selon l'une quelconque des revendications 1 à 8, **caractérisée en ce qu'**au moins l'une desdites formations de support (24) s'étend à la manière d'un porte-à-faux à partir d'au moins l'une desdites parties annulaires (20a, 20b).

10. Prothèse selon l'une quelconque des revendications 1 à 9, **caractérisée en ce qu'**au moins l'une desdites formations de support (24) a une configuration conique.

11. Prothèse selon la revendication 10, **caractérisée en ce qu'**au moins l'une desdites formations de support (24) a une configuration conique avec une largeur qui diminue dans la direction proximale-vers-distale par rapport au sens d'écoulement libre du sang à travers la prothèse.

12. Prothèse selon l'une quelconque des revendications précédentes, **caractérisée en ce qu'**une structure semblable à une fourche (28) est associée à au moins l'une desdites formations de support (24), ladite structure semblable à une fourche (28) comportant une partie de racine raccordée à l'une (20b) desdites parties annulaires (20a, 20b) et deux griffes qui s'étendent à partir de chaque côté par rapport à ladite au moins une desdites formations de support (24) puis qui se raccordent à l'autre (20a) desdites parties annulaires (20a, 20b).

13. Prothèse selon les revendications 9 et 12 combinées, **caractérisée en ce que** lesdites deux griffes se raccordent à l'autre (20a) desdites parties annulaires (20a, 20b) sur des côtés opposés par rapport à la région à partir de laquelle ladite au moins une desdites formations de support (24) fait saillie à la manière d'un porte-à-faux à partir de ladite autre (20a) desdites parties annulaires (20a, 20b).

14. Prothèse selon l'une quelconque des revendications 1 à 8, **caractérisée en ce qu'**elle comprend au moins une paire de formations de support homologues (24) qui s'étendent à la manière de porte-à-faux l'une vers l'autre en partant respectivement de l'une (20a, 20b) et de l'autre (20b, 20a) desdites parties annulaires (20a, 20b).

15. Prothèse selon la revendication 14, **caractérisée en ce que** lesdites formations de support homologues (24) sont raccordées l'une à l'autre de manière flexible par un élément de support (34).

16. Prothèse selon l'une quelconque des revendications 1 à 10, **caractérisée en ce qu'**elle comprend au moins une paire de formations de support combinées (24) qui s'étendent à la manière de porte-à-faux en partant de l'une (20b) desdites parties annulaires (20a, 20b), lesdites formations de support combinées (24) étant raccordées l'une à l'autre par un élément de support en forme de feston (36) qui peut supporter l'un desdits feuillets de valve (3a, 3b, 3c).

17. Prothèse selon l'une quelconque des revendications précédentes, **caractérisée en ce que** lesdites formations d'ancrage (22) et lesdites formations de support (24) forment des parties différentes d'une structure d'armature unique (2).

18. Prothèse selon l'une quelconque des revendications 1 à 15, **caractérisée en ce que** lesdites formations d'ancrage (22) et lesdites formations de support (24) forment des parties qui sont structurellement différentes les unes des autres.

19. Prothèse selon l'une quelconque des revendications précédentes, **caractérisée en ce que** ladite armature (2) est constituée d'un matériau ré-absorbable, au moins de manière limitée audites parties d'ancrage (22).

20. Prothèse selon l'une quelconque des revendications précédentes, **caractérisée en ce que** lesdites formations d'ancrage (22) sont constituées, au moins en partie, d'un matériau à mémoire de forme.

21. Prothèse selon l'une quelconque des revendications précédentes, **caractérisée en ce que** lesdits feuillets de valve (3a, 3b, 3c) sont constitués de matériaux choisis dans le groupe comprenant des tissus biologiques, des tissus synthétiques, des tissus colonisables et des tissus ré-absorbables.
